# EUROPÄISCHE PATENTSCHRIFT

(11) **EP 0 934 423 B1**
(45) Veröffentlichungstag und Bekanntmachung des Hinweises auf die Patenterteilung: **10.01.2007**
(21) Anmeldenummer: 97947710.6
(22) Anmeldetag: 24.10.1997
(51) Int. Cl.: C12N 15/86, C07K 14/015, C07K 14/075, C12N 7/04, C12N 5/10

(54) **AAV-DNA MIT HELFERVIRUS-SEQUENZEN**
AAV-DNA HELPER VIRUS SEQUENCES
ADN DE VIRUS ASSOCIE AUX ADENOVIRUS COMPORTANT DES SEQUENCES DE VIRUS ASSISTANT

(30) Priorität: 25.10.1996 DE 19644500
(43) Veröffentlichungstag der Anmeldung: 11.08.1999
(73) Patentinhaber: Deutsches Krebsforschungszentrum Stiftung des öffentlichen Rechts, 69120 Heidelberg (DE); TRANSGENE S.A., 67082 Strasbourg Cédex (FR)
(72) Erfinder: KLEINSCHMIDT, Jürgen, D-69245 Bammental (DE); GRIMM, Dirk, D-67059 Ludwigshafen (DE); RITTNER, Karola, F-67000 Strasbourg (FR)
(74) Vertreter: Schüssler, Andrea
(86) Internationale Anmeldenummer: PCT/DE1997/002500
(87) Internationale Veröffentlichungsnummer: WO 1998/018950

(56) Entgegenhaltungen:
- WO-A-95/06743
- WO-A-96/18727
- COLOSI P ET AL: "AAV VECTORS CAN BE EFFICIENTLY PRODUCED WITHOUT HELPER VIRUS" BLOOD, Bd. 86, Nr. 10, SUPPL. 01, 15.November 1995, Seite 627A XP000614696
- CLARK K R ET AL: "CELL LINES FOR THE PRODUCTION OF RECOMBINANT ADENO-ASSOCIATED VIRUS" HUMAN GENE THERAPY, Bd. 6, Nr. 10, 1.Oktober 1995, Seiten 1329-1341, XP000569718
- FLOTTE T R ET AL: "AN IMPROVED SYSTEM FOR PACKAGING RECOMBINANT ADENO-ASSOCIATED VIRUSVECTORS CAPABLE OF IN VIVO TRASNDUCTION" GENE THERAPY, Bd. 2, Nr. 1, 1995, Seiten 29-37, XP000609327

## Beschreibung

Die vorliegende Erfindung betrifft AAV-DNA mit Helfervirus-Sequenzen, die für die Ausbildung von AAV- Viruspartikeln notwending sind solche AAV-DNA enthaltendes System und ihre Verwendung.

AAVs (Adeno-assoziierte Viren) sind einzelsträngige, zur Familie der Parvoviren gehörende DNA-Viren. Für ihre Replikation, d.h. zur Ausbildung von Viruspartikeln, benötigen AAVs Helferviren, insbesondere Adenoviren oder Herpesviren. In Abwesenheit von Helferviren können AAVs in das Wirtszellgenom, insbesondere an einer spezifischen Stelle von Chromosom 19, integrieren.

Das Genom von AAVs ist linear und weist eine Länge von ca. 4680 Nukleotiden auf. Es umfaßt zwei Leserahmen, die für ein strukturelles und ein nicht-strukturelles Gen kodieren. Das strukturelle Gen wird mit cap-Gen bezeichnet. Dieses steht unter der Kontrolle des P40-Promotors und kodiert für drei Capsid-Proteine. Das nicht-strukturelle Gen wird mit rep-Gen bezeichnet und kodiert für die Rep-Proteine, Rep 78, Rep 68, Rep 52 und Rep 40. Die beiden ersteren werden unter der Kontrolle des P5 Promotors exprimiert, während die Expression von Rep 52 und Rep 40 unter der Kontrolle des P19 Promotors steht. Die Funktionen der Rep-Proteine liegen u.a. in der Regulation der Replikation und Transkription des AAV-Genoms.

Es hat sich nun gezeigt, daß Präparationen von rekombinanten (r)AAV-Viruspartikeln häufig mit Helferviren, z.B. Adenoviren oder Herpesviren, kontaminiert sind. Diese Kontamination stellt eine erhebliche Limitierung der Verwendung von rAAV-Viruspartikeln für die Gentherapie dar. Bemühungen, die Helferviren durch CsCl-Dichtegradientenzentrifigation oder Filtrationsverfahren zu entfernen, waren bisher wenig erfolgreich, insbesondere umfassen diese Verfahren Schritte, die sich bei den Kosten und in der Ausbeute nachteilig bemerkbar machen.

Aus WO95/06743 ist ein rekombinantes Adenovirus (AdAAV) bekannt, bei dem die AAV-Gene "rep-lip-cap" anstelle der E1-Region von Adenovirus vorliegen.

Aus Blood, Vol. 86, P. 627a, Zusammenfassung 2496 (1995) ist ein Verfahren zur Herstellung von AAV bekannt, bei dem Adenovirus E1a- und E1b-Gene exprimierende Zellen mit einem den AAV-Vektor und Helfersequenzen enthaltenden Plasmid und Kombinationen aus Adenovirus early Gene enthaltenden Plasmiden kotransfiziert werden.

Der vorliegenden Erfindung liegt somit die Aufgabe zugrunde, ein Mittel bereitzustellen, mit dem rAAV-Viruspartikel ohne Kontamination mit Helferviren bereitgestellt werden können.

Erfindungsgemäß wird dies durch die Gegenstände in den Patentansprüchen erreicht.

Gegenstand der vorliegenden Erfindung ist somit eine AAV-DNA mit Helfervirus-Sequenzen, die für die Ausbildung von AAV-Viruspartikeln notwendig sind wobei die AAV-DNA unter DSM 11248 am 18. Oktober 1996 hinterlegt worden ist.

Die vorliegende Erfindung beruht auf der Erkenntnis des Anmelders, daß durch eine erfindungsgemäße AAV-DNA ein in Zellen gemeinsam vorliegender, eine Fremd-DNA enthaltender rAAV-Vektor zur Ausbildung von rAAV-Viruspartikeln veranlaßt wird, ohne daß hierfür Helferviren zugegeben werden müssen.

Der Ausdruck "rAAV-Vektor" umfaßt jegliches AAV-Viruspartikel und dessen DNA, die eine Fremd-DNA, außer einer solchen eines Helfervirus, enthalten können, welche für die Ausbildung von AAV-Viruspartikeln notwendig ist.

Der Ausdruck "Fremd-DNA" betrifft mit vorstehender Ausnahme jegliche DNA, die in einem AAV-Vektor integriert sein kann. Die Fremd-DNA kann nicht-kodierend oder kodierend sein. In ersterem Fall kann sie ein Regulator-Element der DNA-Replikation und/oder Transkription sein. In letzterem Fall ist es günstig, wenn die Fremd-DNA exprimierbar ist, wobei es besonders vorteilhaft ist, wenn die Expression unter der Kontrolle eines induzierbaren Promotors, wie eines gewebespezifischen Promotors, steht. Ferner kann die Fremd-DNA für ein diagnostisches und/oder therapeutisches Protein kodieren. Beispiele eines therapeutischen Proteins sind Tumornekrosefaktor, Plasmaproteine und Rezeptoren. Desweiteren kann die Fremd-DNA an beliebiger Stelle des AAV-Vektors inseriert sein.

Eine erfindungsgemäße AAV-DNA kann durch übliche Verfahren hergestellt werden. Ergänzend wird auf Sambrook, J. et al., Molecular Cloning, A Laboratory Handbook (Band 1-3), Cold Spring Harbour, New York, (1989) verwiesen. Es wird auf die Herstellung der erfindungsgemäßen AAV-DNA pTG 9585 in Beispiel 1 verwiesen. Diese AAV-DNA umfaßt als Helfervirussequenzen die vollständige Adenovirus 5-Sequenz mit Ausnahme der E1-Region. pTG 9585 wurde bei der DSM, Braunschweig, als Plasmid pTG9585 unter der Nummer DSM 11248 am 18. Okt. 1996 hinterlegt. Weiterhin ist eine erfindungsgemäße AAV-DNA bevorzugt, die sich von pTG 9585 darin unterscheidet, daß sie eine Deletion im Strukturgen L1 der Adenovirus 5-Sequenz, insbesondere im Bereich der Nukleotide 16614-18669, aufweist. Diese AAV-DNA wird mit pTG 9585 Δ 16614-18669 bezeichnet. Desweiteren ist eine erfindungsgemäße AAV-DNA bevorzugt, die sich von pTG 9585 darin unterscheidet, daß sie zwei Deletionen von insgesamt 18323 Basenpaaren aufweist, wobei die eine Deletion große Teile der Adenovirus-Kapsidgene und die andere Deletion die E3-Region von Adenovirus betrifft. Diese AAV-DNA wird mit pDG bezeichnet und wurde bei der DSM als Plasmid pDG unter der Nummer DSM 11817 am 15. Oktober 1997 hinterlegt.

Ein weiterer Gegenstand der vorliegenden Erfindung ist ein System aus vorstehenden Elementen, d.h. einer AAV-DNA, einem rAAV-Vektor und gegebenenfalls einer Zelle. Die AAV-DNA und/oder die Zelle stellen hinsichtlich der AAV-Sequenzen des rAAV-Vektors eine Komplementation dar. Der Ausdruck "Zelle" betrifft jegliche Zelle, insbesondere Säugetierzelle, welche die Aufnahme und Vermehrung von AAV erlauben.

Mit der vorliegenden Erfindung ist es möglich, rAAV-Viruspartikel-Präparationen bereitzustellen, ohne daß Helferviren verwendet werden müssen. Die rAAV-Viruspartikel-Präparationen sind somit auch Helfervirus frei. Dies zeigt sich insbesondere bei Verwendung der erfindungsgemäßen AAV-DNA pDG. Ferner zeichnen sich die rAAV-Viruspartikel-Präparationen dadurch aus, daß sie keinen AAV-Wildtyp enthalten. Sie stellen ebenfalls einen Gegenstand der vorliegenden Erfindung dar.

Erfindungsgemäße rAAV-Viruspartikel-Präparationen eignen sich bestens zur Transduktion von Zellen. Günstig kann es sein, wenn die Präparationen vor ihrer Verwendung mit einer DNase behandelt werden, wodurch freie AAV-DNA abgebaut wird. Als Zellen kommen jegliche Zellen in Frage, die im oder isoliert aus einem Körper vorliegen. Mit der vorliegenden Erfindung ist es somit möglich, Maßnahmen für eine ex vivo und in vivo Gentherapie zu ergreifen.

Erläuterung der Zeichnung
- Fig. 1: zeigt die Klonierungsstrategie zum Erhalt der erfindungsgemäßen AAV-DNA pTG9585

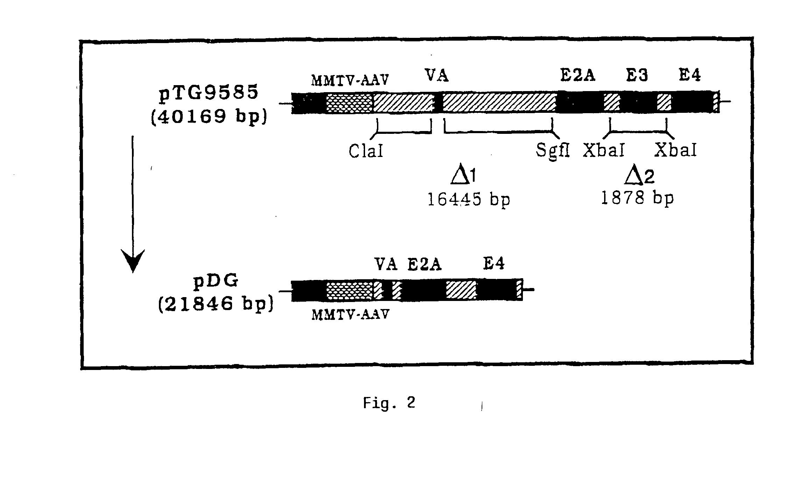

- Fig. 2: zeigt die Klonierungsstrategie zum Erhalt der erfindungsgemäßen AAV-DNA pDG

Die Erfindung wird durch die nachfolgenden Beispiele erläutert.

### Beispiel 1 : Herstellung der erfindungsgemäßen AAV-DNA pTG9585

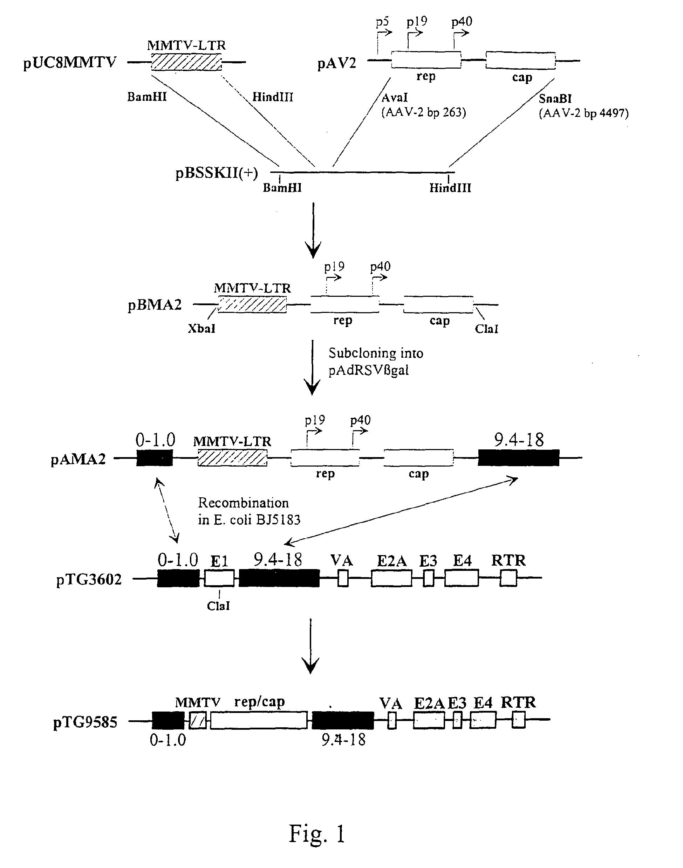

Die Klonierungsstrategie zum Erhalt von pTG9585 ist in Fig. 1 gezeigt. Es wird ein MMTV-LTR-Fragment aus PUC8MMTV (Fasel et al., (1982), EMBO J. 1, S. 3-7) in die multiple Klonierungsstelle des Plasmids pBSSKII(+) (Fa. Stratagene) eingefügt. In dieses Plasmid werden dann mit Hilfe eines synthetischen Oligonukleotidadaptors 4235 bp AAV2-Sequenz aus pAV2 (Gene 23,65-73 (1983)) inseriert (AAV2-Basenpaare 263-4497), welche das vollständige rep- und cap-Gen sowie die AAV2-Promotoren p19 und p40 enthalten. Im resultierenden Plasmid pBMA2 ist somit der AAV2-Promotor p5, der die Expression der Rep78 bzw. Rep68-Proteine steuert, durch den MMTV-Promotor ersetzt. Die komplette Expressionskassette bestehend aus dem MMTV-LTR und dem AAV2 rep- und cap-Gen, wird dann an die Stelle des RSV-βgal-Fragments in den Vektor pAdRSVβgal (J. Clin. Invest. 90, 625-630) eingefügt. Im so entstandenen Plasmid pAMA2 wird das MMTV-AAV2-Fragment beiderseitig von adenoviralen Sequenzen (5': 0-1,0 map units; 3': 9,4-18 map units) flankiert.

Mittels homologer Rekombination (Chartier et al., J. Virol. 70, S. 4805-4810 (1996)) wird das MMTV-AAV2-Fragment aus pAMA2 in das Plasmid pTG3602 (vgl. Chartier et al. vorstehend) eingefügt. Das resultierende Plasmid pTG9585 enthält somit die vollständige Adenovirus 5-Sequenz mit Ausnahme der E1-Region, die durch das MMTV-AAV2-Fragment substituiert ist. pTG9585 stellt eine erfindungsgemäße AAV-DNA dar.

### Beispiel 2: Herstellung der erfindungsgemäßen AAV-DNA pTG9585 Δ 16614-18669

In die in Beispiel 1 hergestellte AAV-DNA pTG9585 wird durch Restriktionsverdau mit Rsrll eine Deletion der Nukleotide 10983-18670 (die Zahlen beziehen sich auf die Adenovirus 5-Sequenz) eingeführt. Danach wird ein Subfragment mit den Nukleotiden 10963-16613 wieder in das deletierte DNA-Molekül eingesetzt. Die Deletion umfaßt somit einen Bereich von 2056 bp (Nukleotide 16614-18669) aus dem Strukturgen L1 von Adenovirus 5. Es wird die erfindungsgemäße AAV-DNA pTG9585 Δ 16614-18669 erhalten.

### Beispiel 3: Herstellung der erfindungsgemäßen AAV-DNA pDG

In die in Beispiel 1 hergestellte AAV-DNA pTG9585 wird durch Restriktionsverdau mit Clal und Sgfl eine Deletion der Nukleotide 5528 - 23677 eingeführt. Diese Deletion (18149 Basenpaare) umfaßt große Teile der Adenovirus 5-Kapsidgene und die für die Bildung von rAAV-Viruspartikeln wichtige VA-Region. Dieser Region (1704 Basenpaare) wird dem verbliebenen Clal/Sgfl-Fragment von pTG 9585 wieder zugeführt. Hierzu wird die VA-Region von Adenovirus 5 mittels PCR amplifiziert und mit 5'- bzw. 3'-Enden versehen, die zu Clal bzw. Sgfl kompatibel sind, so daß ein VA-Fragment erhalten wird, das mit dem vorstehenden Clal/Sgfl-Fragment ligiert werden kann. Es wird eine AAV-DNA pTG 9585 erhalten, die eine Deletion von 16445 Basenpaaren aufweist. Diese AAV-DNA wird pTG 9585 Δ 16445 bezeichnet.

In pTG 9585 Δ 16445 wird eine weitere Deletion eingeführt. Diese betrifft die Adenovirus 5-E3-Region und stellt sich als ein 18-78 Basenpaare umfassendes Xbal-Fragment (30827-32705) dar. Hierzu wird pTG 9585 einem Bgll-Verdau unterzogen und ein Bgll-Fragment (27097 - 37445), das die Adenovirus 5-E3-Region umfaßt, isoliert und in pBSSKII (Stratagene) kloniert. Das erhaltene DNA-Molekül wird einem Xbal-Verdau unterzogen, wodurch das vorstehende Xbal-Fragment abgetrennt werden kann. Das restliche DNA-Molekül wird religiert und nach einem Bgll-Verdau wird das erhaltene Bgll-Fragment, dem die Adenovirus 5-E3-Region fehlt, durch homologe Rekombination in pTG 9585 Δ 16445 eingeführt. Es wird die erfindungsgemäße AAV-DNA pDG erhalten.

## Patentansprüche

1. AAV-DNA mit Helfervirus-Sequenzen, die für die Ausbildung von AAV-Viruspartikeln notwendig sind, wobei die AAV-DNA unter DSM 11248 am 18. Oktober 1996 hinterlegt worden ist.

2. AAV-DNA nach Anspruch 1, wobei die AAV-DNA eine Deletion im Strukturgen L1 der Adenovirus 5-Sequenz aufweist.

3. AAV-DNA nach Anspruch 2, wobei die AAV-DNA unter DSM 11817 am 15. Oktober 1997 hinterlegt worden ist.

4. System, umfassend eine AAV-DNA nach einem der Ansprüche 1 - 3, einen rAAV-Vektor und gegebenenfalls eine Zelle.

5. Verwendung einer AAV-DNA mit Helfervirus-Sequenzen, die für die Ausbildung von AAV-Viruspartikein notwendig sind, zur Herstellung einer rAAV-Viruspartikel-Präparation, die nicht mit Helferviren kontaminiert ist.

6. Verwendung eines Systems, umfassend eine AAV-DNA mit Helfervirus-Sequenzen, die für die Ausbildung von AAV-Viruspartikein notwendig sind, einen rAAV-Vektor und gegebenenfalls eine Zelle, zur Herstellung einer rAAV-Viruspartikel-Präparation, die nicht mit Helferviren kontaminiert ist.

7. Verwendung nach Anspruch 5 oder 6, wobei die Helfervirus-Sequenzen von Herpesvirus stammen.

8. Verwendung nach Anspruch 5 oder 6, wobei die Helfervirus-Sequenzen von Adenovirus stammen.

9. Verwendung nach Anspruch 8, wobei das Adenovirus Adenovirus 5 ist.

10. Verwendung nach einem der Ansprüche 6, 8 oder 9, wobei die AAV-DNA unter DSM 11248 am 18. Oktober 1996 hinterlegt worden ist.

11. Verwendung nach Anspruch 10, wobei die AAV-DNA eine Deletion im Strukturgen L1 der Adenovirus 5-Sequenz aufweist.

12. Verwendung nach Anspruch 11, wobei die AAV-DNA unter DSM 11817 am 15. Oktober 1997 hinterlegt worden ist.

## Claims

1. AAV DNA having helper virus sequences which are necessary for developing AAV viral particles, wherein the AAV DNA was deposited under DSM 11248 on October 18, 1996.

2. The AAV DNA according to claim 1, wherein the AAV DNA comprises a deletion in the structural gene L1 of the adenovirus 5 sequence.

3. The AAV DNA according to claim 2, wherein the AAV DNA was deposited under DSM 11817 on October 15, 1997.

4. A system comprising an AAV DNA according to any one of claims 1 to 3, an rAAV vector and optionally a cell.

5. Use of an AAV DNA having helper virus sequences which are necessary for developing AAV viral particles, for the production of an rAAV viral particle preparation which is not contaminated with helper viruses.

6. Use of a system comprising an AAV DNA having helper virus sequences, which are necessary for developing AAV viral particles, an rAAV vector and optionally a cell,: for the production of an rAAV viral particle preparation which is not contaminated with helper viruses.

7. Use according to claim 5 or 6, wherein the helper virus sequences originate from herpesvirus.

8. Use according to claim 5 or 6, wherein the helper virus sequences originate from adenovirus.

9. Use according to claim 8, wherein the adenovirus is adenovirus 5.

10. Use according to any one of claims 6, 8 or 9, wherein the AAV DNA was deposited under DSM 11248 on October 18, 1996.

11. Use according to claim 10, wherein the AAV DNA comprises a deletion in the structural gene L1 of the adenovirus 5 sequence.

12. Use according to claim 11, wherein the AAV DNA was deposited under DSM 11817 on October 15, 1997.

## Revendications

1. ADN de virus associé aux adénovirus comportant des séquences de virus assistant qui sont nécessaires à la formation des parties de virus adénovirus dans lequel l'ADN d'adénovirus a été déposé le 18 octobre 1996 sous la désignation DSM 11248.

2. ADN de virus associé aux adénovirus dans lequel l'ADN d'adénovirus comporte une délétion dans la structure du gène L1 de la séquence 5 de l'adénovirus.

3. ADN de virus associé aux adénovirus selon la revendication 2, dans lequel l'ADN d'adénovirus a été déposé le 15 octobre 1997 sous la désignation DSM 11817.

4. Système incluant l'ADN de virus associé aux adénovirus selon l'une des revendications 1 à 3, un vecteur de virus associé aux r-adénovirus et le cas échéant une cellule.

5. Utilisation d'un ADN de virus associé aux adénovirus comportant des séquences de virus assistant qui sont nécessaires à la formation de parties de virus adénovirus, pour la fabrication d'une préparation de parties de virus r-adénovirus, qui n'est pas contaminée avec des virus assistants.

6. Utilisation d'un système comprenant un ADN de virus associé aux adénovirus comportant des séquences de virus assistant qui sont nécessaires à la formation de parties de virus adénovirus, un vecteur de r-adénovirus et le cas échéant une cellule, pour la fabrication d'une préparation de parties de virus r-adénovirus, qui n'est pas contaminée avec des virus assistants.

7. Utilisation selon la revendication 5 ou 6, dans laquelle les séquences de virus assistant proviennent des virus de l'herpes.

8. Utilisation selon la revendication 5 ou 6, dans laquelle les séquences de virus assistant proviennent d'adénovirus.

9. Utilisation selon la revendication 8, dans laquelle l'adénovirus est l'adénovirus 5.

10. Utilisation selon l'une des revendications 6, 8 ou 9, dans laquelle l'ADN de virus associé aux adénovirus a été déposé sous la désignation DSM 11248 le 18 octobre 1996.

11. Utilisation selon la revendication 10, dans laquelle l'ADN de virus associé aux adénovirus possède une délétion dans la structure du gène L1 de la séquence 5 de l'adénovirus.

12. Utilisation selon la revendication 11, dans laquelle l'ADN de virus associé aux adénovirus a été déposé sous la désignation DSM 11817 le 15 octobre 1997.
